# EUROPÄISCHE PATENTSCHRIFT

(11) **EP 3 484 338 B1**
(45) Veröffentlichungstag und Bekanntmachung des Hinweises auf die Patenterteilung: **14.02.2024**
(21) Anmeldenummer: 17740713.7
(22) Anmeldetag: 10.07.2017
(51) Int. Cl.: A61B 1/00, A61B 1/06, A61B 1/313, G01S 17/89, G02B 23/24

(54) **ENDOSKOPISCHE VORRICHTUNG UND VERFAHREN ZUR ENDOSKOPISCHEN UNTERSUCHUNG**
ENDOSCOPIC APPARATUS AND METHOD FOR ENDOSCOPIC EXAMINATION
DISPOSITIF ENDOSCOPIQUE ET PROCÉDÉ D'EXAMEN ENDOSCOPIQUE

(30) Priorität: 14.07.2016 DE 102016113000
(43) Veröffentlichungstag der Anmeldung: 22.05.2019
(73) Patentinhaber: Aesculap AG, 78532 Tuttlingen (DE)
(72) Erfinder: MAAS, Hans-Gerd, 01187 Dresden (DE); CONEN, Niklas Paul, 49716 Meppen (DE); LUHMANN, Thomas, 26203 Wardenburg (DE)
(74) Vertreter: Hoeger, Stellrecht & Partner Patentanwälte mbB
(86) Internationale Anmeldenummer: PCT/EP2017/067195
(87) Internationale Veröffentlichungsnummer: WO 2018/011106

(56) Entgegenhaltungen:
- DE-A1-102008 018 636
- DE-A1-102011 087 357
- DE-A1-102016 109 173
- US-A1- 2009 268 010
- US-A1- 2011 228 049
- US-A1- 2013 331 648

## Beschreibung

Die Erfindung betrifft eine bildgebende endoskopische Vorrichtung, insbesondere für medizinische Anwendungen, umfassend ein Endoskop mit einem in einen Untersuchungsgegenstand einführbaren Schaft und eine Datenverarbeitungseinheit sowie optische Abbildungseinheiten zum Bereitstellen von Bilddatensätzen für die Datenverarbeitungseinheit.

Außerdem betrifft die Erfindung ein Verfahren zur endoskopischen Untersuchung eines technischen Untersuchungsgegenstandes, bei dem ein Schaft einer endoskopischen Vorrichtung in den Untersuchungsgegenstand eingeführt wird und ein Objekt im Untersuchungsgegenstand abgebildet wird, wobei einer Datenverarbeitungseinheit Bilddatensätze bereitgestellt werden. Offenbart ist ferner ein Verfahren zur endoskopischen Untersuchung für medizinische Anwendungen.

Die vorliegende Erfindung wird nachfolgend insbesondere mit Bezug auf eine medizinische Anwendung beschrieben, sie ist jedoch nicht auf dieses Anwendungsgebiet beschränkt. Endoskopische Untersuchungen können zum Beispiel auch im Bereich der Fertigung oder der Wartung technischer Gegenstände durchgeführt werden. Als diesbezügliches Anwendungsbeispiel wird die endoskopische Untersuchung von Gasturbinen genannt, wie sie in der WO 2013/045108 A1 beschrieben ist.

Bei medizinischen Anwendungen wird der Schaft des Endoskops in den (menschlichen oder tierischen) Körper als Untersuchungsgegenstand eingeführt, um Objekte wie zum Beispiel innere Organe in Hohlräumen, den chirurgischen Eingriff unterstützend, abzubilden. Abgebildet werden können auch chirurgische Instrumente, derer sich der Operateur während des Eingriffes bedient. Bekannt ist in diesem Zusammenhang der Einsatz von Stereoendoskopen, um dem Operateur eine räumliche Darstellung eines Objektes zu vermitteln.

In der WO 2006/005061 A2 ist eine Vorrichtung beschrieben, bei der drei optische Abbildungseinheiten zum Einsatz kommen. Dem Operateur kann mittels zweier Abbildungseinheiten ein Stereobild an einer Anzeigeeinheit dargestellt werden. Es besteht ferner die Möglichkeit, ein zusätzliches Bild unter Einsatz einer dritten Abbildungseinheit zu erstellen und beispielsweise als Insert im Stereobild darzustellen, um dem Operateur zusätzliche Informationen bereitzustellen.

Die US 2009/0268010 A1 beschreibt eine Vorrichtung und ein Verfahren zur endoskopischen Untersuchung. Mittels zweier Bildsensoren können Einzelbilder eines Objektes im Untersuchungsraum für eine stereoskopische Darstellung erstellt werden. Zusätzlich kann ein weiterer Bildsensor vorgesehen sein, mit dem ein Fluoreszenzbild des Objektes erstellt wird. An einer stereoskopischen Anzeige kann das Fluoreszenzbild wahlweise angezeigt und dabei der stereoskopischen Darstellung überlagert werden. Eine Ausführungsform sieht vor, dass ein Paar von Fluoreszenzbildern erstellt wird, die jeweils wahlweise dem Stereobilddatensatz überlagert werden können.

In der US 2013/0331648 A1 ist ein Verfahren und eine Vorrichtung für endoskopische Untersuchungen offenbart. Es besteht die Möglichkeit, eine dreidimensionale Repräsentation eines Inneren einer Körperkavität zu erstellen. Der Operateur kann digital in der dreidimensionalen Repräsentation navigieren, um das Innere der Körperkavität von unterschiedlichen Punkten zu betrachten. Dreidimensionale Darstellungen können "geöffnet" und planar in Abhängigkeit von der Zeit dargestellt werden.

Aus der DE 10 2008 018 636 A1 sind Vorrichtungen und Verfahren zur endoskopischen 3D-Erfassung bekannt.

Die DE 10 2011 087 357 A1 beschreibt ein Verfahren zum Aktualisieren von präoperativ aufgenommenen 3D-Bilddaten eines Körpers hinsichtlich der aktuellen operativen Situation. Dabei werden intraoperativ exo- oder endoskopische 3D-Daten bevorzugt intrakorporal erfasst und mit diesen die 3D-Bilddaten korrigiert.

Die US 2011/0228049 A1 betrifft Systeme und Verfahren zur 3D-Darstellung von Bildern.

Aufgabe der vorliegenden Erfindung ist es, eine endoskopische Vorrichtung und ein Verfahren zur endoskopischen Untersuchung bereitzustellen, mit der bzw. dem ergänzende Informationen zur umfassenderen Untersuchung des Untersuchungsgegenstandes gewonnen werden können.

Diese Aufgabe wird durch eine erfindungsgemäße endoskopische Vorrichtung mit den Merkmalen von Anspruch 1 gelöst.

Bei der erfindungsgemäßen Vorrichtung ist vorgesehen, dass zumindest drei Abbildungseinheiten zum Einsatz kommen, wobei bei einer vorteilhaften Ausführungsform auch vier oder mehr Abbildungseinheiten vorgesehen sein können. Am Schaft distal angeordnet sind Abbildungselemente, in deren Sichtfeld ein abzubildendes Objekt liegt. Von den Abbildungselementen gebündeltes Licht kann auf die Bildsensoren übertragen werden, welche im Schaft angeordnet oder in einem extern zum Untersuchungsgegenstand positionierten Gehäuse angeordnet sein können. Die Datenverarbeitungseinheit untersucht die Bilddatensätze auf korrespondierende (sogenannte homologe) Bildpunkte, wobei die Bilddatensätze von drei oder mehr Bildsensoren und bevorzugt von allen Bildsensoren berücksichtigt werden. Anders als bei den aus dem Stand der Technik bekannten Vorrichtungen besteht erfindungsgemäß die Möglichkeit, dass eine 3D-Rekonstruktion der beobachteten Szene möglich ist. Durch das Vorsehen von drei oder mehr Bilddatensätzen können eventuelle Mehrdeutigkeiten und Ungenauigkeiten, wie sie bekanntermaßen bei der Auswertung von Stereo-Bilddatensätzen auftreten können, weitgehend eliminiert werden. Mittels der Vorrichtung kann dadurch ein 3D-Oberflächendatensatz abgebildeter Objekte erstellt werden, der sich durch höhere Genauigkeit auszeichnet. Der 3D-Oberflächendatensatz kann der weiteren endoskopischen Untersuchung zugrunde gelegt werden und wird beispielsweise für zeitabhängige Erfassungen der Änderung der Position und/oder der Form eines Objektes oder mehrerer berücksichtigt, worauf später noch eingegangen wird.

Die erfindungsgemäße Vorrichtung eignet sich in besonderer Weise für medizinische endoskopische Untersuchungen. Diese Untersuchungen stehen vor der Herausforderung, dass endoskopische Vorrichtungen der Kompaktheit bedürfen, damit die Invasivität für den Patienten möglichst gering gehalten werden kann (beispielsweise sind etwaige zusätzliche Inzisionen zu vermeiden), insbesondere unter Berücksichtigung der beengten Platzverhältnisse im Körper. Als besonders schwierig bei der Erfassung, Rekonstruktion und Identifizierung von körpereigenen Strukturen erweist sich die Tatsache, dass diese überwiegend texturarm ausgestaltet sind und daher nur wenige strukturelle, bildverarbeitungstechnisch einfach zu analysierende Eigenschaften aufweisen. Als schwierig erweist es sich auch, dass von körpereigenen Strukturen aufgrund der schwach ausgeprägten Struktur und anhaftender Flüssigkeit wie Wasser oder Blut Reflexionen ausgehen, die in den Bilddatensätzen nur schwer analysiert werden können. Fördernd für an sich unerwünschte Reflexionen ist dabei eine dennoch erforderliche Beleuchtung im Körperinneren. Aufgrund der räumlichen Einschränkung besteht außerdem in der Praxis das Problem, dass Abbildungselemente mit großen Aperturen eingesetzt werden, welche Verzerrungen in den Bilddatensätzen bedingen und nur eine geringe Tiefenschärfe ermöglichen, wobei diese auch durch den geringen Basisabstand der Abbildungselemente distal am Schaft bedingt ist. Die vorliegende Erfindung ermöglicht es durch die Nutzung von mindestens drei Bilddatensätzen, Mehrdeutigkeiten zu einem hohen Grad zu eliminieren und dadurch insgesamt zuverlässigere Informationen über abgebildete Objekte zu erlangen.

Der 3D-Oberflächendatensatz kann aus einer sogenannten "Punktewolke" aus einer endlichen Zahl von Punkten erstellt werden oder umfasst eine solche endliche Zahl von Punkten, die anhand der identifizierten korrespondierenden Bildpunkte ermittelt werden.

Zur Identifikation und Bestimmung von korrespondierenden Bildpunkten in den drei oder mehr Bilddatensätzen kann der Fachmann auf ihm bekannte Algorithmen des Mehrbild-Matchings zurückgreifen, beispielsweise nach dem Kernlinienschnittverfahren.

Günstig ist es, wenn die Datenverarbeitungseinheit die korrespondierenden Bildpunkte zur Erstellung des 3D-Datensatzes in Echtzeit ermittelt, beispielsweise in Intervallen von wenigen Sekunden und vorzugsweise im Millisekundenbereich. Bei einer Visualisierung des 3D-Oberflächendatensatzes an einer Anzeigeeinheit kann ein Objekt auf diese Weise gewissermaßen in Echtzeit dargestellt werden.

Vorteilhaft ist es, dass Positions- und/oder Formänderungen des Objekts von der Vorrichtung zeitabhängig ermittelbar sind, wodurch das Objekt, zumindest teilweise, getrackt werden kann. Eine Bewegung des Objektes mit einer Änderung in Lage und/oder Orientierung und/oder eine Formänderung eines oder mehrerer Objekte(s) kann von der Datenverarbeitungseinheit dadurch festgestellt werden, dass aufeinanderfolgende 3D-Datensätze Unterschiede aufweisen, wobei das Objekt oder die Objekte (vorzugsweise simultan) jeweils in den 3D-Datensätzen identifiziert und dadurch zeitabhängig verfolgt werden kann/können. Hierdurch ergibt sich ein großer Nutzen für den Anwender. Im Bereich medizinischer Endoskopie lassen sich dadurch beispielsweise pulsierende Organe erkennen und tracken.

Es kann vorgesehen sein, dass die Datenverarbeitungseinheit anhand von zwei Bilddatensätzen einen Stereobilddatensatz erstellt, der auf korrespondierende Bildpunkte mit zumindest einem weiteren Bilddatensatz untersucht wird.

Weiter kann vorgesehen sein, dass je zwei Bilddatensätze stereoskopisch miteinander kombiniert und mit einem weiteren Bilddatensatz verglichen werden. Dementsprechend ist es bei einer bevorzugten Ausführungsform von Vorteil, wenn die Datenverarbeitungseinheit von je zwei Bilddatensätzen einen Stereobilddatensatz erstellt, der auf korrespondierende Bildpunkte mit einem jeweiligen weiteren Bilddatensatz untersucht wird.

Es kann vorgesehen sein, dass die Vorrichtung eine mit der Datenverarbeitungseinheit gekoppelte Anzeigeeinheit umfasst.

Günstig ist es, wenn die Datenverarbeitungseinheit anhand von zwei Bilddatensätzen ein Stereobild des Objektes erstellt und an der Anzeigeeinheit darstellt. Dem Anwender, beispielsweise dem Operateur, kann ein intuitiv erfassbares Stereobild zur Führung des Endoskops im Untersuchungsgegenstand angezeigt werden.

Alternativ oder ergänzend ist es günstig, wenn die Datenverarbeitungseinheit ein Bild des 3D-Datensatzes der Anzeigeeinheit insbesondere zeitabhängig darstellt. Der von der Datenverarbeitungseinheit erstellte 3D-(Oberflächen-) Datensatz mit dem anhand der Bildinformation rekonstruierten Objekt kann dem Anwender wertvolle zusätzliche Information während des endoskopischen Eingriffs liefern. Fehlfarbendarstellung zur Hervorhebung interessanter Eigenschaften des Objektes ist möglich. Eine Navigation im dargestellten 3D-Datensatz für den Anwender ist vorteilhafterweise möglich, um sich das Objekt von unterschiedlichen Seiten betrachten zu können, ohne zu diesem Zweck das Endoskop führen zu müssen.

Die Abbildungselemente können bei einer vorteilhaften Ausführungsform kollinear am Schaft angeordnet sein. In Draufsicht auf das distale Ende des Schaftes können die Abbildungselemente nebeneinanderliegend positioniert sein, beispielsweise äquidistant. Jeweilige von den Abbildungselementen definierte Achsen sind dabei entlang einer durch sie verlaufenden Geraden angeordnet und/oder paarweise parallel zueinander ausgerichtet.

Bei einer andersartigen vorteilhaften Ausführungsform ist es günstig, wenn eines der Abbildungselemente symmetrisch bezüglich einer von zwei weiteren anderen Abbildungselementen gebildeten Basis am Schaft angeordnet ist. Beispielsweise bilden zwei Abbildungselemente eine Basis eines stereoskopischen Systems, wobei ein drittes Abbildungselement symmetrisch zur Basis positioniert ist.

Günstigerweise sind die Abbildungselemente, insbesondere bei der zuletzt erwähnten vorteilhaften Ausführungsform, in regelmäßiger Anordnung am Schaft angeordnet, beispielsweise gemäß einem gleichschenkligen und insbesondere gleichseitigen Dreieck, jeweils bezogen auf eine Draufsicht auf den Schaft in proximaler Richtung. Bei regelmäßiger Anordnung der Abbildungselemente, beispielsweise von drei Abbildungselementen, gemäß einem gleichseitigen Dreieck, kann eine möglichst kompakte Bauform des Endoskops erzielt werden.

Als günstig erweist es sich, wenn zumindest zwei Abbildungselemente in planarer Anordnung zueinander angeordnet sind. Hierunter kann der Fachmann vorliegend insbesondere verstehen, dass optische Achsen der Abbildungselemente parallel zueinander ausgerichtet sind. Optische Ebenen der Abbildungselemente, insbesondere Linsenebenen, fallen vorzugsweise zusammen.

Alternativ oder ergänzend kann vorgesehen sein, dass zumindest zwei Bildsensoren in planarer Anordnung zueinander angeordnet sind. Darunter kann vorliegend insbesondere verstanden werden, dass von den Bildsensoren gebildete Ebenen zusammenfallen oder parallel zueinander angeordnet sein können.

Bei einer vorteilhaften Ausführungsform ist vorgesehen, dass jeweils alle Abbildungselemente und/oder alle Bildsensoren in planarer Anordnung zueinander angeordnet sind.

Die optischen Abbildungseigenschaften der Abbildungselemente (Apertur, Brennweite etc.) sind vorzugsweise identisch. Die Abbildungseigenschaften der Abbildungseinheiten insgesamt können identisch sein.

Bei einer vorteilhaften Ausführungsform ist es günstig, wenn die Bildsensoren im Schaft angeordnet sind und über Signalleitungen mit der außerhalb des Untersuchungsgegenstandes positionierten Datenverarbeitungseinheit gekoppelt sind. Es besteht insbesondere die Möglichkeit, dass ein sogenanntes "Chip-on-the-Tip"-Endoskop vorgesehen ist.

Bei einer andersartigen vorteilhaften Ausführungsform ist vorgesehen, dass die Abbildungselemente über im Schaft geführte Lichtleitelemente mit den Bildsensoren gekoppelt sind, die außerhalb des Untersuchungsgegenstandes in einem Gehäuse angeordnet sind. Objekte werden über die Abbildungselemente in die Lichtleitelemente abgebildet und über diese auf die extern zum Untersuchungsgegenstand angeordneten Bildsensoren.

Als günstig erweist es sich, wenn die Vorrichtung eine Beleuchtungseinheit umfasst mit mindestens einem in den Untersuchungsgegenstand einführbaren Leuchtelement. Dies gibt die Möglichkeit, die Szene zu beleuchten und von Objekten Abbildungen höherer Qualität zu erstellen.

Vorteilhafterweise ist eine Mehrzahl von Leuchtelementen vorgesehen. Die Leuchtelemente können vorzugsweise frei zueinander positionierbar sein und/oder günstigerweise unabhängig voneinander aktivierbar oder deaktivierbar, damit die Vorrichtung für die Bedienperson möglichst vielseitig einsetzbar ist.

Vorzugsweise ist jeder Abbildungseinheit ein Leuchtelement zugeordnet, wobei die Anzahl der Leuchtelemente gleich der Anzahl der Abbildungseinheiten sein kann.

Günstig ist es, wenn das Leuchtelement mindestens einen im Schaft geführten Lichtleiter umfasst oder bildet. Die Integration des Lichtleiters in den Schaft ermöglicht es, ein ergänzend zum Schaft in den Untersuchungsgegenstand einzuführendes Leuchtelement einzusparen. Der Bedienperson wird die Handhabung der Vorrichtung dadurch vereinfacht, dass mit dem Einwirken auf das Endoskop zugleich der Lichtleiter bewegt wird. Ist der Lichtleiter im Hinblick auf eine vorteilhafte Ausleuchtung des Sichtfeldes der Abbildungselemente angepasst, kann dadurch eine optische Abbildung hoher Güte erzielt werden. Der Lichtleiter ist oder umfasst beispielsweise ein Bündel von im Schaft geführten Glasfasern.

Bei einer Mehrzahl von Lichtleitern ist vorzugsweise vorgesehen, dass die Lichtleiter symmetrisch zueinander und/oder symmetrisch relativ zu den Abbildungselementen im Schaft und insbesondere distal am Schaft, bezogen auf eine Draufsicht, angeordnet sind. Wünschenswert ist dabei eine möglichst homogene Ausleuchtung des Sichtfeldes der Abbildungselemente.

Die Lichtleiter können zur Minimierung von Reflexionen vorteilhafterweise radial außenseitig bezüglich der Abbildungselemente angeordnet sein, bezogen auf eine Achse des Schaftes. Darunter kann vorliegend insbesondere verstanden werden, dass die Lichtleiter von der Schaftachse radial weiter beabstandet sind als Achsen der Abbildungselemente.

Der Schaft kann starr oder flexibel sein. Bei einem flexiblen Schaft kann eine feststellbare Flexibilität vorgesehen sein.

Nachfolgend wird auf unterschiedliche Ausgestaltungen der Bildsensoren eingegangen, wie sie in unterschiedlichen Ausführungsformen vorhanden sein können. Denkbar ist grundsätzlich ein Austausch von Bildsensoren, wobei in diesem Fall deren Anordnung in einem extern zum Untersuchungsgegenstand angeordneten Gehäuse wie vorstehend erläutert von Vorteil sein kann.

Vorgesehen sein kann, dass zumindest zwei der drei oder mehr Bildsensoren sich hinsichtlich spektraler Empfindlichkeit und/oder Auflösung voneinander unterscheiden.

Beispielsweise kann die spektrale Empfindlichkeit zumindest eines Bildsensors im Infrarotbereich, im Bereich des sichtbaren Spektrums oder im Ultraviolettbereich liegen. Durch Einsatz von IR- oder UV-fähigen Bildsensoren können der Bedienperson Informationen bereitgestellt werden, die bei herkömmlichen Endoskopen mit einer Empfindlichkeit im Bereich des sichtbaren Spektrums, auch Stereoendoskopen, nicht verfügbar sind.

Bei einer vorteilhaften Umsetzung der Vorrichtung können zumindest zwei der drei oder mehr Bildsensoren hinsichtlich der spektralen Empfindlichkeit und/oder Auflösung identisch ausgestaltet sein.

Beispielsweise sind zwei Bildsensoren Monochrom-Sensoren in Bezug auf Graustufen oder einen Farbwert (monochrome Farbe). Zwei Monochrom-Bildsensoren können zum Beispiel mit einem Farbbildsensor (beispielsweise RGB) kombiniert werden. Die Bilddatensätze der Monochrom-Sensoren können zur stereoskopischen Betrachtung mit hoher Auflösung verwendet werden. Für hochaufgelöste dreidimensionale Farbdarstellungen können die Monochrom-Bilddatensätze mit Hilfe des Farbbilddatensatzes eingefärbt werden. Hierbei kann beispielsweise ein Pansharpening-Verfahren eingesetzt werden. Der Farbbilddatensatz kann alternativ oder ergänzend als Kontrollbild und/oder zur Erkennung von Ausreißern in den Monochrom-Bilddatensätzen genutzt werden.

Bei einer andersartigen vorteilhaften Ausführungsform sind beispielsweise zwei Farbbildsensoren vorgesehen (beispielsweise RGB) und ein dritter Bildsensor, dessen spektrale Empfindlichkeit in einem anderen Wellenlängenbereich liegt, wie beispielsweise im Infraroten oder im Ultravioletten. Eine mögliche geringere Auflösung der Bilddatensätze in dem anderen Spektralbereich kann, beispielsweise mittels Pansharpening, mit den Farbbilddatensätzen kompensiert werden.

Wie erwähnt, können zwei Bildsensoren Monochrom-Sensoren sein und der dritte Bildsensor ein Farbbildsensor.

Bei einer andersartigen vorteilhaften Ausführungsform kann vorgesehen sein, dass zumindest ein Bildsensor ein Time-of-Flight-Sensor ist oder einen solchen umfasst, der einen Abstandsbilddatensatz bereitstellt, und dass die Datenverarbeitungseinheit anhand des Abstandsbilddatensatzes eine Abstandsinformation zum Abgleich mit einem aus anderen Bilddatensätzen gewonnenen Stereobilddatensatz ermittelt. Näherungswerte von Oberflächen von Objekten für das Stereomatching können dadurch bestimmt werden.

Günstig ist es, wenn anhand der Datenverarbeitungseinheit eine Kontrolle der Abbildungseigenschaften der Abbildungseinheiten durchführbar ist und bei Abweichung von einem Sollzustand vorzugsweise ein diesbezüglicher Hinweis ausgebbar ist. Die Datenverarbeitungseinheit kann beispielsweise korrespondierende Bildpunkte zeitabhängig überwachen und damit gewissermaßen eine fortlaufende Kontrolle der Bilddatensätze vornehmen. Dies ist vorteilhaft, wenn äußere Einflüsse oder zum Beispiel die Erwärmung des Endoskops zur Veränderung der Orientierung der Abbildungseinheiten führen und sich dadurch die Abbildungseigenschaften verändern. Durch Bereitstellen eines Hinweises kann eine Bedienperson auf diesen Umstand hingewiesen werden. Alternativ oder ergänzend ist es günstig, wenn von der Datenverarbeitungseinheit eine automatische Kompensation der veränderten Abbildungseigenschaften durchgeführt werden kann.

Die Vorrichtung kann mindestens ein Werkzeug zum Handhaben im Untersuchungsgegenstand aufweisen. Das Werkzeug, beispielsweise ein chirurgisches Instrument, umfasst günstigerweise eine Kennzeichnung, die von der Datenverarbeitungseinheit in den Bilddatensätzen erkennbar ist zur Identifikation des Werkzeuges. Das Werkzeug kann dadurch insbesondere hinsichtlich seiner Position nach Lage und/oder Orientierung zeitabhängig verfolgt werden (getrackt). Eine Darstellung des so getrackten Werkzeuges in einem visualisierten 3D-Datensatz eines Objektes ist zur Unterstützung der Bedienperson beim Eingriff von Vorteil.

Die eingangs genannte Aufgabe wird durch ein erfindungsgemäßes Verfahren zur endoskopischen Untersuchung eines Untersuchungsgegenstandes mit den Merkmalen von Anspruch 15 gelöst.

Die Vorteile, die bereits im Zusammenhang mit der Erläuterung der erfindungsgemäßen Vorrichtung erwähnt wurden, können unter Einsatz des Verfahrens ebenfalls erzielt werden. Diesbezüglich kann auf die voranstehenden Ausführungen verwiesen werden.

Vorteilhafte Ausführungsbeispiele des Verfahrens ergeben sich durch vorteilhafte Ausführungsformen der Vorrichtung, so dass auch diesbezüglich auf die voranstehenden Erläuterungen verwiesen werden kann.

Die nachfolgende Beschreibung bevorzugter Ausführungsformen der Erfindung dient im Zusammenhang mit der Zeichnung der näheren Erläuterung der Erfindung. Mit der erfindungsgemäßen Vorrichtung kann das erfindungsgemäße Verfahren durchgeführt werden. Es zeigen:
- Figur 1:: eine erfindungsgemäße endoskopische Vorrichtung für eine medizinische Anwendung an einem Patienten durch einen Operateur;
- Figur 2:: schematisch die Vorrichtung aus Figur 1;
- Figur 3:: eine Darstellung eines distalen Endes des Schaftes eines Endoskops der Vorrichtung, in Richtung des Pfeiles "A" in Figur 2;
- Figur 4:: eine Darstellung entsprechend Figur 3 bei einer andersartigen Ausgestaltung des Endoskops; und
- Figur 5:: ein Stereobild eines chirurgischen Instrumentes und eines Objektes (Organ o. ä.) im Körper eines untersuchten Patienten.

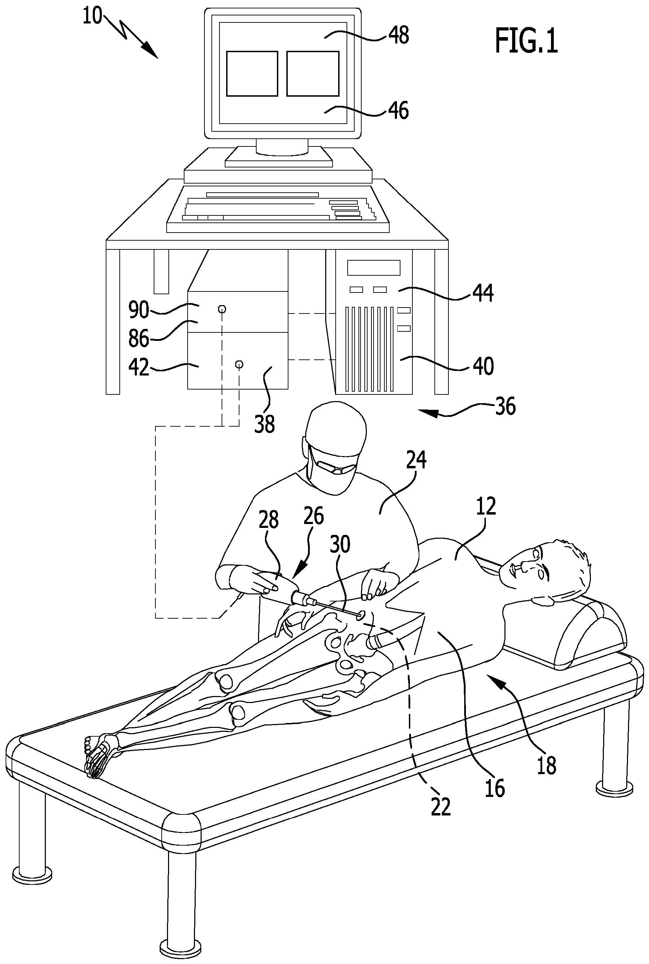

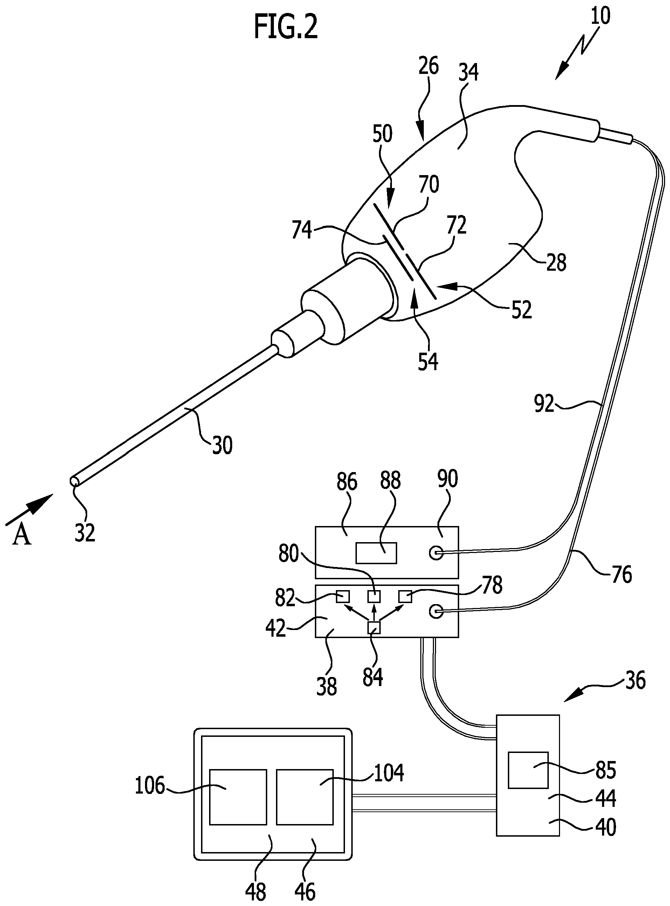

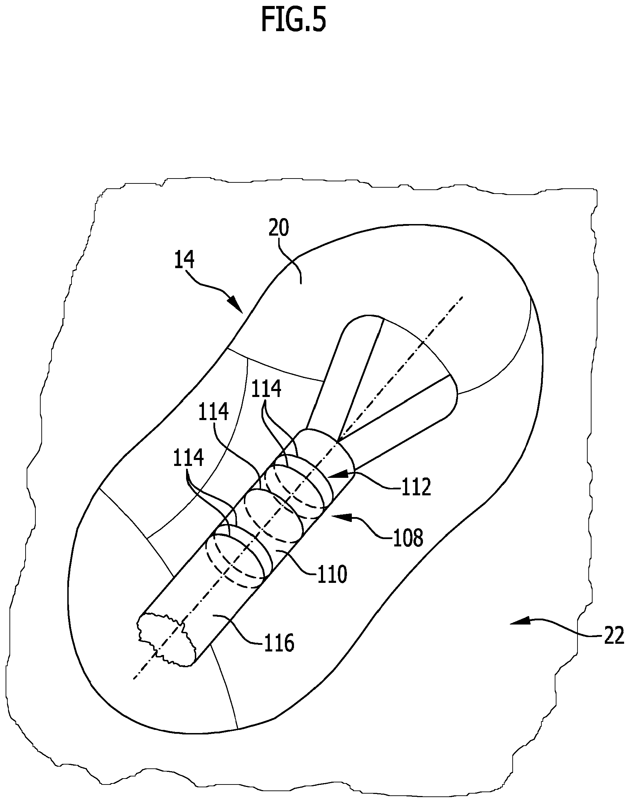

Die Figuren 1 und 2 zeigen schematisch eine mit dem Bezugszeichen 10 belegte vorteilhafte Ausführungsform einer erfindungsgemäßen bildgebenden endoskopischen Vorrichtung. Die Vorrichtung 10 kommt zur endoskopischen Untersuchung eines Untersuchungsgegenstandes 12 zum Einsatz, um in diesem vorhandene Objekte zu untersuchen, von denen Figur 5 beispielhaft ein Objekt 14 zeigt. Es können mehrere abzubildende Objekte vorgesehen sein und vorliegend simultan untersucht werden.

Die Anwendung der Vorrichtung 10 ist am Beispiel eines operativen Eingriffs dargestellt, wobei die vorliegende Erfindung nicht auf medizinische Anwendungen beschränkt ist. Endoskopische Vorrichtungen können beispielsweise auch zur Kontrolle von technischen Vorrichtungen bei der Herstellung und der Wartung eingesetzt werden.

Wie nachfolgend erläutert umfasst die Vorrichtung 10 drei Abbildungseinheiten. Andersartige Ausführungsformen können, wie bereits erwähnt, mehr als drei Abbildungseinheiten umfassen.

Bei der vorliegenden beispielhaften Anwendung ist der Untersuchungsgegenstand 12 dementsprechend der Körper 16 eines Patienten 18, und das Objekt 14 ist beispielsweise ein zu untersuchendes Organ 20 im Bauchraum 22. Die Bedienperson der Vorrichtung 10 ist ein Operateur 24.

Die Vorrichtung 10 umfasst ein vom Operateur 24 handgeführtes Endoskop 26 mit einem Griffelement 28 und einem daran gehaltenen, zumindest teilweise in den Körper 16 einführbaren Schaft 30. Der Schaft 30 weist ein distales Ende 32 auf, das bei bestimmungsgemäßer Anwendung des Endoskopes 26 an der vom Operateur 24 abgewandten Seite angeordnet ist. Das Griffelement 28 umfasst oder bildet ein Gehäuse 34.

Der Schaft 30 ist vorliegend starr ausgestaltet, könnte jedoch auch flexibel sein. Alternativ oder ergänzend kann vorgesehen sein, dass der Schaft 30 am Griffelement 28 positionsveränderlich gehalten ist.

Weiter umfasst die Vorrichtung 10 eine Datenverarbeitungseinheit 36, die im vorliegenden Fall zwei signalwirksam miteinander gekoppelte und in Gehäusen 38, 40 angeordnete Bestandteile umfasst. Im Gehäuse 38 ist eine Auswerteeinheit 42 der Datenverarbeitungseinheit 36 aufgenommen und im Gehäuse 40 eine Recheneinheit 44. Denkbar ist selbstverständlich auch, dass die Datenverarbeitungseinheit 36 ein gemeinsames Gehäuse aufweist, das sowohl die Auswerteeinheit 42 als auch die mit dieser gekoppelte Recheneinheit 44 aufnimmt.

Die Datenverarbeitungseinheit 36 ist mit einer Anzeigeeinheit 46 gekoppelt, die insbesondere eine Bildanzeige 48 umfasst.

Die Vorrichtung 10 umfasst vorliegend drei optische Abbildungseinheiten 50, 52 und 54. Jede Abbildungseinheit 50, 52, 54 umfasst ein am distalen Ende 32 im Schaft 30 gefasstes Abbildungselement 56, 58 bzw. 60 auf. Die Abbildungselemente 56, 58, 60 können vorzugsweise identisch ausgestaltet sein und sind beispielsweise in Form von Linsen ausgebildet.

Die Abbildungselemente 56, 58, 60 sind in planarer Anordnung zueinander am distalen Ende 32 des Schaftes 30 angeordnet, wobei von ihnen jeweils definierte Achsen 62, 64 bzw. 66 parallel zueinander und parallel zu einer vom Schaft 30 definierten Achse 68 verlaufen. Linsenebenen der Abbildungselemente 56, 58 und 60 fallen zusammen.

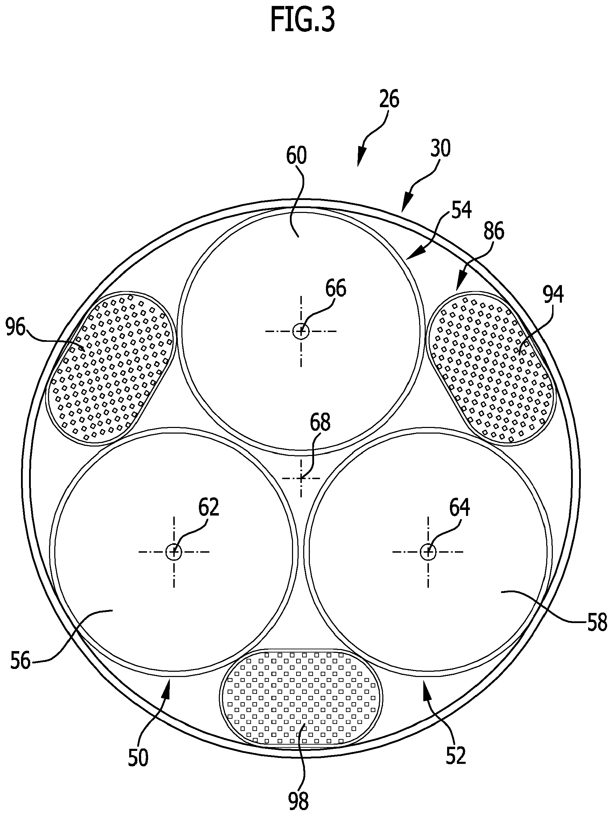

Die Abbildungselemente 56, 58 und 60 sind symmetrisch zueinander gemäß einem gleichseitigen Dreieck am Schaft 30 positioniert (Figur 3, die axial in proximaler Richtung auf das distale Ende 32 des Schaftes 30 blickt).

Jedes Abbildungselement 56, 58 und 60 definiert ein in der Zeichnung nicht dargestelltes Sichtfeld, in dem Bereiche des Bauchraumes 22 und insbesondere das Organ 20 angeordnet sein können. Objekte im jeweiligen Sichtfeld eines Abbildungselementes 56, 58, 60 werden auf Bildsensoren 70, 72 bzw. 74 der Abbildungseinheiten 50, 52 bzw. 54 abgebildet. Jedem Abbildungselement 56, 58, 60 ist ein Bildsensor 70, 72 bzw. 74 zugeordnet (d. h. 56-70, 58-72, und 60-74).

Von den Abbildungselementen 56, 58, 60 gesammeltes Licht wird durch im Schaft 30 geführte, in der Zeichnung nicht dargestellte Lichtleitelemente bis zum Gehäuse 34 des Griffelementes 28 geführt, in dem die Bildsensoren 70, 72, 74 angeordnet sind. Es können weitere Abbildungselemente vorgesehen sein (nicht gezeigt), um Licht jeweils auf einen der Bildsensoren 70, 72, 74 abzubilden.

Bei einer andersartigen vorteilhaften Ausführungsform kann vorgesehen sein, dass Bildsensoren direkt im Schaft 30 positioniert sind, beispielsweise unmittelbar proximal der Abbildungselemente 56, 58, 60, wodurch Lichtleitelemente eingespart werden können.

Die Bildsensoren 70, 72, 74 sind über eine Signalleitung 76 mit der Auswerteeinheit 42 gekoppelt. Ein jeweiliger von einem Bildsensor 70, 72, 74 bereitgestellter Bilddatensatz 78, 80 bzw. 82 kann von einem Auswerteglied 84 der Auswerteeinheit 42 vorverarbeitet werden (schematisch in Figur 2 dargestellt). Die Bilddatensätze 78, 80 und 82 und/oder vorverarbeitete Informationen können einem Rechenglied 85 der Recheneinheit 44 zugeführt werden.

Insgesamt besteht aufgrund der Ausbildung und Programmierung der Datenverarbeitungseinheit 36 die Möglichkeit, die Bilddatensätze 78, 80 und 82 der Bildsensoren 70, 72 bzw. 74 zu analysieren.

Die Vorrichtung 10 umfasst eine Beleuchtungseinheit 86 zum Beleuchten der Szene im Körperinneren, um die Abbildungseigenschaften der Vorrichtung 10 zu verbessern. Die Beleuchtungseinheit 86 umfasst eine Lichtquelle 88, die vorliegend in einem Gehäuse 90 extern zum Endoskop 26 aufgenommen ist. Eine Lichtleitung 92 ist vom Gehäuse 90 zum Gehäuse 34 des Endoskops 26 geführt. An die Lichtleitung 92 sind drei Leuchtelemente 94, 96 und 98 angekoppelt, die vorliegend als Lichtleitelemente in Gestalt von Glasfaserbündeln ausgestaltet sind.

Die Leuchtelemente 94, 96, 98 sind vom Gehäuse 34 durch den Schaft 30 geführt und erstrecken sich bis zum distalen Ende 32.

Zumindest im Bereich des distalen Endes 32 sind die Leuchtelemente 94, 96, 98 symmetrisch zueinander gemäß einem gleichseitigen Dreieck (bezogen auf eine proximale Blickrichtung auf das distale Ende 32) angeordnet. Weiter besteht eine Symmetrie der Anordnung der Leuchtelemente 94, 96, 98 in Bezug auf die Abbildungselemente 56, 58 und 60. Je ein Leuchtelement 94, 96, 98 ist einem der Abbildungselemente 56, 58 bzw. 60 bezüglich der Achse 68 des Schaftes 30 diametral gegenüberliegend angeordnet.

Auf diese Weise wird eine möglichst gleichmäßige Ausleuchtung der Szene im Körperinneren ermöglicht. Die Anordnung der Leuchtelemente 94, 96, 98 distal radial außenseitig bezüglich der Abbildungselemente 56, 58 und 60 erweist sich als vorteilhaft zur Vermeidung von Reflexionen an den an den zu visualisierenden Objekten.

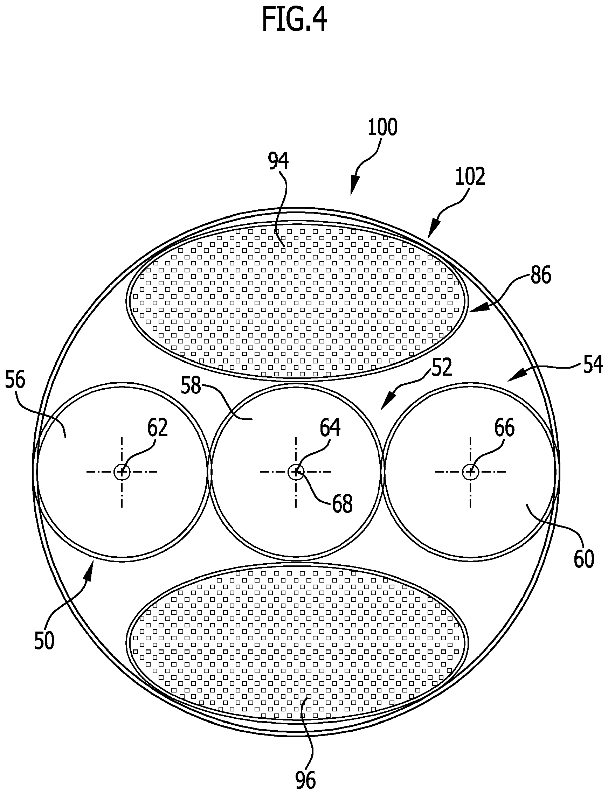

Bei einer andersartigen vorteilhaften Ausführungsform einer erfindungsgemäßen Vorrichtung kann ein zum Endoskop 26 unterschiedlich ausgestaltetes Endoskop 100 vorgesehen sein, dessen Schaft 102 in Figur 4 in einer der Figur 3 entsprechenden Weise dargestellt ist.

Beim Endoskop 100 sind die Abbildungselemente 56, 58, 60 kollinear positioniert, wobei deren Achsen 62, 64 bzw. 66 parallel zueinander verlaufen. Die Achse des mittleren Abbildungselementes fällt mit der Achse 68 des Schaftes 102 zusammen.

Die Beleuchtungseinheit 86 umfasst bei der andersartigen Vorrichtung zwei Leuchtelemente 94, 96, die seitlich neben der Dreier-Anordnung der Abbildungselemente 56, 58 und 60 positioniert sind.

Wie bereits erläutert können die Bildsensoren 70, 72 und 74 unterschiedlich ausgestaltet sein. Beispielsweise sind die Bildsensoren 70 und 72 identisch und als Monochrom-Sensoren und speziell als Graustufen-Sensoren ausgestaltet. Die Bildsensoren 70, 72 können dementsprechend eine vergleichsweise hohe Auflösung erzielen.

Der Bildsensor 74 kann sich bei der Vorrichtung 10 hinsichtlich der Auflösung und/oder spektralen Empfindlichkeit von den Bildsensoren 70, 72 unterscheiden. Vorliegend ist der Bildsensor 74 beispielsweise ein Farbbildsensor zur Farbbilddarstellung, zum Beispiel im RGB-Format.

Die Datenverarbeitungseinheit 36 ist so ausgebildet und programmiert, dass sie anhand der Bilddatensätze 78, 80 der Bildsensoren 70 bzw. 72 ein Stereobild 104 erstellt. Das Stereobild 104 kann an der Anzeigeeinheit 46 dargestellt werden und zeigt dem Operateur 24 die Szene im Bauchraum 22, um ihm die Führung des Endoskopes 26 zu erleichtern.

Darüber hinaus ist die Datenverarbeitungseinheit 36 so ausgebildet und programmiert, dass sie die Bilddatensätze 78, 80 und 82 auf korrespondierende (sogenannte homologe) Bildpunkte analysiert und korrespondierende Bildpunkte in den Bilddatensätzen 78, 80 und 82 ermittelt. Auf diese Weise ist es der Datenverarbeitungseinheit 36 mit hoher Präzision möglich, etwaige Mehrdeutigkeiten, wie sie im Falle von nur zwei Bilddatensätzen auftreten können, unter Berücksichtigung eines dritten Bilddatensatzes auszuschließen.

Anhand der Bilddatensätze 78, 80 und 82 kann die Datenverarbeitungseinheit 36 einen 3D-Oberflächendatensatz abgebildeter Objekte erstellen, beispielsweise des Organs 20. Ein 3D-Bild 106 des 3D-Datensatzes kann an der Anzeigeeinheit 46 dargestellt werden. Es besteht auch die Möglichkeit, das 3D-Bild 106 mit dem Stereobild 104 zu überlagern.

Die Analyse der Bilddatensätze 78, 80 und 82 kann insbesondere in Echtzeit im Sub-Sekunden-Intervall durchgeführt werden. Die fortlaufende Analyse der Bilddatensätze 78, 80, 82 erlaubt es der Datenverarbeitungseinheit 36, Positions- und/oder Formänderungen des Organs 20 zeitabhängig zu ermitteln. Das Organ 20 kann dementsprechend von der Datenverarbeitungseinheit 36 getrackt werden, wobei durch fortlaufende Analyse der Bilddatensätze 78, 80, 82 jeweils die Oberfläche des Organs 20 nahezu in Echtzeit rekonstruiert wird. Dies steigert den Nutzen und die Anwenderfreundlichkeit der Vorrichtung 10 für den Operateur 24 immens. Insbesondere verfügt der Operateur 24 über zusätzliche Informationen, die bei herkömmlichen endoskopischen Vorrichtungen nicht verfügbar sind.

Bei der Berücksichtigung der drei Abbildungseinheiten 50, 52 und 54 lassen sich Schwierigkeiten beheben, die beim Einsatz herkömmlicher Technik zur Visualisierung, auch Stereovisualisierung, im medizinischen Umfeld auftreten. Wie bereits erwähnt können Mehrdeutigkeiten weitgehend eliminiert werden. Dies ermöglicht die Oberflächenrekonstruktion von Bereichen des Organs 20, die homogen oder arm an Textur sind. Reflexionen am untersuchten Organ 20 sind weniger störend, weil durch die zusätzlichen Bildinformationen die Suche nach korrespondierenden Bildpunkten in den Bilddatensätzen 78, 80 und 82 erleichtert wird.

Zudem ergibt sich durch die Integration aller Abbildungselemente 56, 58 und 60 sowie ergänzend der Leuchtelemente 94, 96 und 98 in demselben Schaft 30 eine sehr kompakte Bauform. Eine Untersuchung des Patienten 18 ist mit geringer Invasivität möglich.

Die Vorrichtung 10 kann ferner mindestens ein Werkzeug 108 aufweisen, das vorliegend als chirurgisches Instrument 110 ausgestaltet ist. Am Instrument 110 kann eine Kennzeichnung 112 vorgesehen sein. Vorliegend umfasst die Kennzeichnung 112 eine Mehrzahl von koaxialen Ringen 114, die axial voneinander beabstandet an einem Schaft 116 des Instrumentes 110 angeordnet sind.

Die Datenverarbeitungseinheit 36 kann das Instrument 110 anhand der Kennzeichnung 112 identifizieren und ebenso wie das Organ 20 als beobachtetes Objekt tracken.

### Bezugszeichenliste:

- 10: Vorrichtung
- 12: Untersuchungsgegenstand
- 14: Objekt
- 16: Körper
- 18: Patient
- 20: Organ
- 22: Bauchraum
- 24: Operateur
- 26: Endoskop
- 28: Griffelement
- 30: Schaft
- 32: distales Ende
- 34: Gehäuse
- 36: Datenverarbeitungseinheit
- 38: Gehäuse
- 40: Gehäuse
- 42: Auswerteeinheit
- 44: Recheneinheit
- 46: Anzeigeeinheit
- 48: Bildanzeige
- 50, 52, 54: Abbildungseinheit
- 56, 58, 60: Abbildungselement
- 62, 64, 66: Achse
- 68: Achse
- 70, 72, 74: Bildsensor
- 76: Signalleitung
- 78, 80, 82: Bilddatensatz
- 84: Auswerteglied
- 85: Rechenglied
- 86: Beleuchtungseinheit
- 88: Lichtquelle
- 90: Gehäuse
- 92: Lichtleitung
- 94, 96, 98: Leuchtelement
- 100: Endoskop
- 102: Schaft
- 104: Stereobild
- 106: 3D-Bild
- 108: Werkzeug
- 110: Instrument
- 112: Kennzeichnung
- 114: Ring
- 116: Schaft

## Patentansprüche

1. Endoskopische Vorrichtung, insbesondere für medizinische Anwendungen, umfassend ein Endoskop (26; 100) mit einem in einen Untersuchungsgegenstand (12) einführbaren Schaft (30; 102) und eine Datenverarbeitungseinheit (36), drei oder mehr optische Abbildungseinheiten (50, 52, 54) mit jeweiligen, distal am Schaft (30; 102) angeordneten Abbildungselementen (56, 58, 60) und diesen zugeordneten Bildsensoren (70, 72, 74) zum Bereitstellen von drei oder mehr Bilddatensätzen (78, 80, 82) für die Datenverarbeitungseinheit (36),
**dadurch gekennzeichnet, dass**
die Datenverarbeitungseinheit (36) ausgebildet und programmiert ist:
- jeden der drei oder mehr Bilddatensätze (78, 80, 82) zu analysieren und korrespondierende Bildpunkte in jedem der drei oder mehr Bilddatensätze (78, 80, 82) zu ermitteln;
- anhand der in den drei oder mehr Bilddatensätzen (78, 80, 82) ermittelten korrespondierenden Bildpunkte einen 3D-Oberflächendatensatz eines von den Abbildungseinheiten (50, 52, 54) abgebildeten Objektes (14) im Untersuchungsgegenstand (12) zu erstellen;
- Unterschiede von aufeinanderfolgenden 3D-Oberflächendatensätzen festzustellen, unter Identifikation des Objektes (14) jeweils im 3D-Oberflächendatensatz;
- zeitabhängig Positions- und/oder Formänderungen des Objekts (14) in den aufeinanderfolgenden 3D-Oberflächendatensätzen zu ermitteln, um das Objekt (14), zumindest teilweise, zu tracken.

2. Vorrichtung nach Anspruch 1, wobei die Datenverarbeitungseinheit (36) die korrespondierenden Bildpunkte zur Erstellung des 3D-Datensatzes in Echtzeit ermittelt.

3. Vorrichtung nach einem der voranstehenden Ansprüche, wobei die Datenverarbeitungseinheit (36) anhand von zwei Bilddatensätzen (78, 80) einen Stereobilddatensatz erstellt, der auf korrespondierende Bildpunkte mit zumindest einem weiteren Bilddatensatz (82) untersucht wird, insbesondere die Datenverarbeitungseinheit (36) von je zwei Bilddatensätzen einen Stereobilddatensatz erstellt, der auf korrespondierende Bildpunkte mit einem jeweiligen weiteren Bilddatensatz untersucht wird.

4. Vorrichtung nach einem der voranstehenden Ansprüche, wobei die Vorrichtung (10) eine mit der Datenverarbeitungseinheit (36) gekoppelte Anzeigeeinheit (46) umfasst und die Datenverarbeitungseinheit (36) anhand von zwei Bilddatensätzen (78, 80) ein Stereobild (104) des Objektes (14) erstellt und an der Anzeigeeinheit (46) darstellt und/oder die Datenverarbeitungseinheit (36) ein Bild (106) des 3D-Datensatzes an der Anzeigeeinheit (46) darstellt.

5. Vorrichtung nach einem der voranstehenden Ansprüche, wobei die Abbildungselemente (56, 58, 60) kollinear am Schaft (102) angeordnet sind oder eines der Abbildungselemente (56, 58, 60) symmetrisch bezüglich einer von zwei weiteren Abbildungselementen (56, 58, 60) gebildeten Basis am Schaft (30) angeordnet ist.

6. Vorrichtung nach einem der voranstehenden Ansprüche, wobei zumindest eines der Folgenden gilt:
- die Abbildungselemente (56, 58, 60) sind in regelmäßiger Anordnung am Schaft (30) angeordnet, beispielsweise gemäß einem gleichschenkligen und insbesondere gleichseitigen Dreieck;
- zumindest zwei Abbildungselemente (56, 58, 60) sind in planarer Anordnung zueinander angeordnet und/oder zumindest zwei Bildsensoren (70, 72, 74) sind in planarer Anordnung zueinander angeordnet.

7. Vorrichtung nach einem der voranstehenden Ansprüche, wobei die Bildsensoren im Schaft angeordnet sind und über Signalleitungen mit der außerhalb des Untersuchungsgegentandes positionierten Datenverarbeitungseinheit gekoppelt sind oder die Abbildungselemente (56, 58, 60) über im Schaft (30; 102) geführte Lichtleitelemente mit den Bildsensoren (70, 72, 74) gekoppelt sind, die außerhalb des Untersuchungsgegenstandes (12) in einem Gehäuse (34) angeordnet sind.

8. Vorrichtung nach einem der voranstehenden Ansprüche, wobei die Vorrichtung (10) eine Beleuchtungseinheit (86) umfasst mit mindestens einem in den Untersuchungsgegenstand (12) einführbaren Leuchtelement (94, 96, 98) und/oder der Schaft (30; 102) starr oder flexibel ist.

9. Vorrichtung nach Anspruch 8, wobei eine Mehrzahl von Leuchtelementen (94, 96, 98) vorgesehen ist, wobei vorzugsweise jeder Abbildungseinheit (50, 52, 54) ein Leuchtelement (94, 96, 98) zugeordnet ist, und/oder das Leuchtelement (94, 96, 98) mindestens einen im Schaft (30; 102) geführten Lichtleiter umfasst oder bildet.

10. Vorrichtung nach Anspruch 9, wobei die Lichtleiter symmetrisch zueinander und/oder symmetrisch relativ zu den Abbildungselementen (56, 58, 60) im Schaft (30; 102) und insbesondere distal am Schaft (30; 102) angeordnet sind.

11. Vorrichtung nach Anspruch 10, wobei die Lichtleiter radial außenseitig bezüglich der Abbildungselemente (56, 58, 60) angeordnet sind, bezogen auf eine Achse (68) des Schaftes (30; 102).

12. Vorrichtung nach einem der voranstehenden Ansprüche, wobei zumindest eines der Folgenden gilt:
- zumindest zwei der drei oder mehr Bildsensoren (70, 72, 74) unterscheiden sich hinsichtlich spektraler Empfindlichkeit und/oder Auflösung voneinander;
- die spektrale Empfindlichkeit zumindest eines Bildsensors (70, 72, 74) liegt im Infrarotbereich, im Bereich des sichtbaren Spektrum oder im Ultraviolettbereich;
- zumindest zwei der drei oder mehr Bildsensoren (70, 72, 74) sind hinsichtlich der Auflösung und/oder der spektralen Empfindlichkeit identisch ausgestaltet, insbesondere als Monochrom-Sensoren oder als Farbbildsensoren;
- zwei Bildsensoren (70, 72, 74) sind Monochrom-Sensoren und der dritte Bildsensor (70, 72, 74) ein Farbbildsensor;
- zumindest ein Bildsensor ist ein Time-of-Flight-Sensor oder umfasst einen solchen, der einen Abstandsbilddatensatz bereitstellt, wobei die Datenverarbeitungseinheit anhand des Abstandsbilddatensatzes eine Abstandsinformation zum Abgleich mit einem aus anderen Bilddatensätzen gewonnenen Stereobilddatensatz ermittelt.

13. Vorrichtung nach einem der voranstehenden Ansprüche, wobei anhand der Datenverarbeitungseinheit (36) eine Kontrolle der Abbildungseigenschaften der Abbildungseinheiten (50, 52, 54) durchführbar ist und bei Abweichung von einem Sollzustand vorzugsweise ein diesbezüglicher Hinweis ausgebbar ist.

14. Vorrichtung nach einem der voranstehenden Ansprüche, wobei die Vorrichtung (10) mindestens ein Werkzeug (108) zum Handhaben im Untersuchungsgegenstand (12) aufweist, das eine Kennzeichnung (112) umfasst, die von der Datenverarbeitungseinheit (36) in den Bilddatensätzen (78, 80, 82) erkennbar ist zur Identifikation des Werkzeuges (108).

15. Verfahren zur endoskopischen Untersuchung eines technischen Untersuchungsgegenstandes, bei dem ein Schaft einer endoskopischen Vorrichtung nach einem der voranstehenden Ansprüche in den Untersuchungsgegenstand eingeführt wird und ein Objekt im Untersuchungsgegenstand abgebildet wird, wobei drei oder mehr optische Abbildungseinheiten mit jeweiligen, distal am Schaft angeordneten Abbildungselementen und diesen zugeordneten Bildsensoren zum Bereitstellen von drei oder mehr Bilddatensätzen für die Datenverarbeitungseinheit vorgesehen sind, wobei die Datenverarbeitungseinheit so ausgebildet und programmiert ist, dass sie:
- jeden der drei oder mehr Bilddatensätze analysiert und korrespondierende Bildpunkte in jedem der drei oder mehr Bilddatensätze ermittelt;
- anhand der in den drei oder mehr Bilddatensätze ermittelten korrespondierenden Bildpunkten einen 3D-Oberflächendatensatz des von den Abbildungseinheiten abgebildeten Objektes erstellt;
- Unterschiede von aufeinanderfolgenden 3D-Oberflächendatensätzen feststellt, unter Identifikation des Objektes jeweils im 3D-Oberflächendatensatz;
- zeitabhängig Positions- und/oder Formänderungen des Objektes in den aufeinanderfolgenden 3D-Oberflächendatensätzen ermittelt und das Objekt, zumindest teilweise, trackt.

## Claims

1. Endoscopic device, in particular for medical applications, comprising an endoscope (26; 100) with a shaft (30; 102) that is introducible into a subject under examination (12), and a data processing unit (36), three or more optical imaging units (50, 52, 54) having respective imaging elements (56, 58, 60) arranged distally on the shaft (30; 102) and image sensors (70, 72, 74) associated therewith for providing three or more image data sets (78, 80, 82) for the data processing unit (36), **characterized in that** the data processing unit (36) is configured and programmed:
- to analyze each of the three or more image data sets (78, 80, 82) and to identify corresponding image points in each of the three or more image data sets (78, 80, 82);
- to generate a 3D surface data set of an object (14) imaged by the imaging units (50, 52, 54) in the subject under examination (12) using the corresponding image points identified in the three or more image data sets (78, 80, 82);
- to determine differences of successive 3D surface data sets by identifying the object (14) in the respective 3D surface data set;
- to determine changes in the position and/or shape of the object (14) in the successive 3D surface data sets as a function of time in order to track the object (14), at least partly.

2. Device in accordance with claim 1, wherein the data processing unit (36) determines the corresponding image points for generating the 3D data set in real time.

3. Device in accordance with any one of the preceding claims, wherein the data processing unit (36) uses two image data sets (78, 80) to generate a stereo image data set, which is examined for corresponding image points in at least one further image data set (82), in particular the data processing unit (36) generates from each two image data sets a stereo image data set, which is examined for corresponding image points in a respective further image data set.

4. Device in accordance with any one of the preceding claims, wherein the device (10) comprises a display unit (46) coupled to the data processing unit (36), and the data processing unit (36) uses two image data sets (78, 80) to generate a stereo image (104) of the object (14) and represents it on the display unit (46), and/or the data processing unit (36) represents an image (106) of the 3D data set on the display unit (46).

5. Device in accordance with any one of the preceding claims, wherein the imaging elements (56, 58, 60) are arranged collinearly on the shaft (102) or one of the imaging elements (56, 58, 60) is arranged on the shaft (30) symmetrically in relation to a base formed by two further imaging elements (56, 58, 60).

6. Device in accordance with any one of the preceding claims, wherein at least one of the following applies:
- the imaging elements (56, 58, 60) are arranged in a regular arrangement on the shaft (30), for example in an isosceles and in particular equilateral triangle;
- at least two imaging elements (56, 58, 60) are arranged in a planar arrangement with one another, and/or at least two image sensors (70, 72, 74) are arranged in a planar arrangement with one another.

7. Device in accordance with any one of the preceding claims, wherein the image sensors are arranged in the shaft and are coupled by way of signal lines to the data processing unit, which is positioned outside the subject under examination or the imaging elements (56, 58, 60) are coupled, by way of light conducting elements guided in the shaft (30; 102), to the image sensors (70, 72, 74), which are arranged in a housing (34), outside the subject under examination (12).

8. Device in accordance with any one of the preceding claims, wherein the device (10) comprises an illuminating unit (86) having at least one illuminating element (94, 96, 98) that is introducible into the subject under examination (12) and/or the shaft (30; 102) is rigid or flexible.

9. Device in accordance with claim 8, wherein a plurality of illuminating elements (94, 96, 98) is provided, wherein preferably an illuminating element (94, 96, 98) is associated with each imaging unit (50, 52, 54) and/or the illuminating element (94, 96, 98) comprises or forms at least one light conductor guided in the shaft (30; 102).

10. Device in accordance with claim 9, wherein the light conductors are arranged symmetrically to one another and/or symmetrically in relation to the imaging elements (56, 58, 60) in the shaft (30; 102) and in particular distally on the shaft (30; 102).

11. Device in accordance with claim 10, **characterized in that** the light conductors are arranged radially to the outside of the imaging elements (56, 58, 60) in relation to an axis (68) of the shaft (30; 102).

12. Device in accordance with any one of the preceding claims, wherein at least one of the following applies:
- at least two of the three or more image sensors (70, 72, 74) differ from one another in respect of their spectral sensitivity and/or resolution;
- the spectral sensitivity of at least one image sensor (70, 72, 74) lies in the infrared range, the range of the visible spectrum, or the ultraviolet range;
- at least two of the three or more image sensors (70, 72, 74) have an identical configuration in respect of their resolution and/or spectral sensitivity, in particular as monochrome sensors or as color sensors;
- two image sensors (70, 72, 74) are monochrome sensors and the third image sensor (70, 72, 74) is a color image sensor;
- at least one image sensor is or comprises a time-of-flight sensor that provides a distance image data set, wherein the data processing unit uses the distance image data set to determine an item of distance information for the purpose of comparison with a stereo image data set obtained from other image data sets.

13. Device in accordance with any one of the preceding claims, wherein, using the data processing unit (36), monitoring of the imaging properties of the imaging units (50, 52, 54) is performable and, in the event of a discrepancy from a setpoint condition, an indication of this fact is preferably outputtable.

14. Device in accordance with any one of the preceding claims, wherein the device (10) has at least one tool (108) for the purpose of manipulation in the subject under examination (12), wherein the tool (108) comprises a coding (112), which is detectable in the image data sets (78, 80, 82) by the data processing unit (36) for the purpose of identifying the tool (108).

15. Method for the endoscopic examination of a technical subject under examination, in which method a shaft of an endoscopic device in accordance with any one of the preceding claims is introduced into the subject under examination and an object in the subject under examination is imaged, wherein three or more optical imaging units having respective imaging elements that are arranged distally on the shaft and image sensors associated therewith are provided for the purpose of providing three or more image data sets for the data processing unit, wherein the data processing unit is configured and programmed such that it:
- analyzes each of the three or more image data sets and identifies corresponding image points in each of the three or more image data sets;
- generates a 3D surface data set of the object imaged by the imaging units using the corresponding image points identified in the three or more image data sets;
- determines differences of successive 3D surface data sets by identifying the object in the respective 3D surface data set;
- determines changes in the position and/or shape of the object in the successive 3D surface data sets as a function of time and tracks the object, at least partly.

## Revendications

1. Dispositif endoscopique, en particulier pour applications médicales, comprenant un endoscope (26; 100) avec une tige (30; 102) qui peut être insérée dans un sujet à examiner (12) et une unité de traitement de données (36), trois unités d'imagerie optique (50, 52, 54) ou plus avec des éléments d'imagerie (56, 58, 60) respectifs disposés de manière distale sur la tige (30; 102) et des capteurs d'images (70, 72, 74) qui leur sont associés pour fournir trois ensembles de données d'image (78, 80, 82) ou plus pour l'unité de traitement de données (36), **caractérisé en ce que** l'unité de traitement de données (36) est conçue et programmée:
- pour analyser chacun des trois ensembles de données d'image (78, 80, 82) ou plus et pour déterminer des points d'image correspondants dans chacun des trois ensembles de données d'image (78, 80, 82) ou plus;
- à l'aide des points d'image correspondants déterminés dans les trois ensembles de données d'image (78, 80, 82) ou plus, créer un ensemble de données de surface 3D d'un objet (14) représenté sous forme d'image par les unités d'imagerie (50, 52, 54) dans le sujet à examiner (12);
- établir des différences d'ensembles de données de surface 3D successifs, avec identification de l'objet (14) dans chaque cas dans l'ensemble de données de surface 3D;
- déterminer des changements de position et/ou de forme en fonction du temps de l'objet (14) dans les ensembles de données de surface 3D successifs pour suivre l'objet (14), au moins partiellement.

2. Dispositif selon la revendication 1, où l'unité de traitement de données (36) détermine les points d'image correspondants pour créer l'ensemble de données 3D en temps réel.

3. Dispositif selon l'une des revendications précédentes, où l'unité de traitement de données (36) crée à l'aide de deux ensembles de données d'image (78, 80) un ensemble de données d'image stéréo qui est examiné pour des points d'image correspondants avec au moins un autre ensemble de données d'image (82), en particulier l'unité de traitement de données (36) crée à partir de deux ensembles de données d'image un ensemble de données d'image stéréo qui est examiné pour des points d'image correspondants avec un autre ensemble de données d'image respectif.

4. Dispositif selon l'une des revendications précédentes, où le dispositif (10) comprend une unité d'affichage (46) couplée à l'unité de traitement de données (36) et l'unité de traitement de données (36) crée une image stéréo (104) de l'objet (14) à l'aide de deux ensembles de données d'image (78, 80) et la représente sur l'unité d'affichage (46) et/ou l'unité de traitement de données (36) représente une image (106) de l'ensemble de données 3D sur l'unité d'affichage (46).

5. Dispositif selon l'une des revendications précédentes, où les éléments d'imagerie (56, 58, 60) sont disposés de manière colinéaire sur la tige (102) ou l'un des éléments d'imagerie (56, 58, 60) est disposé sur la tige (30) de manière symétrique par rapport à une base formée par deux autres éléments d'imagerie (56, 58, 60).

6. Dispositif selon l'une des revendications précédentes, où au moins l'un des éléments suivants s'applique:
- les éléments d'imagerie (56, 58, 60) sont disposés dans une disposition régulière sur la tige (30), par exemple selon un triangle isocèle et en particulier équilatéral;
- au moins deux éléments d'imagerie (56, 58, 60) sont disposés dans une disposition plane les uns par rapport aux autres et/ou au moins deux capteurs d'images (70, 72, 74) sont disposés dans une disposition plane les uns par rapport aux autres.

7. Dispositif selon l'une des revendications précédentes, où les capteurs d'images sont disposés dans la tige et sont couplés via des lignes de signaux à l'unité de traitement de données positionnée à l'extérieur du sujet à examiner ou les éléments d'imagerie (56, 58, 60) sont couplés via des éléments de guidage de lumière insérés dans la tige (30; 102) aux capteurs d'images (70, 72, 74) qui sont disposés dans un boîtier (34) à l'extérieur du sujet à examiner (12).

8. Dispositif selon l'une des revendications précédentes, où le dispositif (10) comprend une unité d'éclairage (86) avec au moins un élément lumineux (94, 96, 98) qui peut être inséré dans le sujet à examiner (12) et/ou la tige (30; 102) est rigide ou flexible.

9. Dispositif selon la revendication 8, où une multiplicité d'éléments lumineux (94, 96, 98) est prévue, où, de préférence, à chaque unité d'imagerie (50, 52, 54) est associé un élément lumineux (94, 96, 98), et/ou l'élément lumineux (94, 96, 98) comprend ou forme au moins un guide de lumière inséré dans la tige (30; 102).

10. Dispositif selon la revendication 9, où les guides de lumière sont disposés symétriquement les uns par rapport aux autres et/ou symétriquement par rapport aux éléments d'imagerie (56, 58, 60) dans la tige (30; 102) et en particulier de manière distale sur la tige (30; 102).

11. Dispositif selon la revendication 10, où les guides de lumière sont disposés radialement du côté l'extérieur par rapport aux éléments d'imagerie (56, 58, 60), par rapport à un axe (68) de la tige (30; 102).

12. Dispositif selon l'une des revendications précédentes, où au moins l'un des éléments suivants s'applique:
- au moins deux des trois capteurs d'images (70, 72, 74) ou plus diffèrent les uns des autres concernant la sensibilité spectrale et/ou la résolution;
- la sensibilité spectrale d'au moins un capteur d'images (70, 72, 74) est située dans le domaine infrarouge, dans le domaine du spectre visible ou dans le domaine ultraviolet;
- au moins deux des trois capteurs d'images (70, 72, 74) ou plus sont conçus de manière identique concernant la résolution et/ou la sensibilité spectrale, en particulier sous forme de capteurs monochromes ou sous forme de capteurs d'images couleur;
- deux capteurs d'images (70, 72, 74) sont des capteurs monochromes et le troisième capteur d'images (70, 72, 74) est un capteur d'images couleur;
- au moins un capteur d'images est un capteur de temps de vol ou comprend un tel capteur, qui fournit un ensemble de données d'image de distance, où l'unité de traitement de données détermine à l'aide de l'ensemble de données d'image de distance des informations de distance pour l'égalisation avec un ensemble de données d'image stéréo obtenu à partir d'autres ensembles de données d'image.

13. Dispositif selon l'une des revendications précédentes, où à l'aide de l'unité de traitement de données (36) un contrôle des propriétés d'imagerie des unités d'imagerie (50, 52, 54) peut être effectué et, en cas d'écart par rapport à un état cible, de préférence une indication à ce sujet peut être émise.

14. Dispositif selon l'une des revendications précédentes, où le dispositif (10) présente au moins un outil (108) destiné à être manipulé dans le sujet à examiner (12), qui comprend un marquage (112) qui peut être reconnu par l'unité de traitement de données (36) dans les ensembles de données d'image (78, 80, 82) pour l'identification de l'outil (108).

15. Procédé pour l'examen endoscopique d'un sujet technique à examiner, où une tige d'un dispositif endoscopique selon l'une des revendications précédentes est introduite dans le sujet à examiner et un objet dans le sujet à examiner est représenté sous forme d'image, où trois unités d'imagerie optique ou plus, avec des éléments d'imagerie respectifs disposés de manière distale sur la tige et des capteurs d'images qui leur sont associés sont prévues pour fournir trois ensembles de données d'image ou plus pour l'unité de traitement de données, où l'unité de traitement de données est conçue et programmée de telle manière qu'elle:
- analyse chacun des trois ensembles de données d'image ou plus et détermine des points d'image correspondants dans chacun des trois ensembles de données d'image ou plus;
- à l'aide des points d'image correspondants déterminés dans les trois ensembles de données d'image ou plus, crée un ensemble de données de surface 3D de l'objet représenté sous forme d'image par les unités d'imagerie;
- établit des différences d'ensembles de données de surface 3D successifs, avec identification de l'objet dans chaque cas dans l'ensemble de données de surface 3D;
- détermine des changements de position et/ou de forme de l'objet en fonction du temps dans les ensembles de données de surface 3D successifs et suit l'objet, au moins partiellement.
